# EUROPEAN PATENT APPLICATION

(11) **EP 4 101 493 A1**
(43) Date of publication of application: **14.12.2022**
(21) Application number: 22176187.7
(22) Date of filing: 30.05.2022
(51) Int. Cl.: A61M 25/00, A61M 25/10

(54) **BALLOON CATHETER AND METHODS OF MANUFACTURING THE SAME**

(30) Priority: 04.06.2021 US 202117339726
(71) Applicant: Medtronic Vascular Inc., Santa Rosa, CA 95403 (US)
(72) Inventor: REID, Mark, Santa Rosa (US); ENGLISH, Andrew, Santa Rosa (US); MCNALLY, Orlagh, Santa Rosa (US); FLANAGAN, Kyle, Santa Rosa (US)
(74) Representative: Zimmermann & Partner Patentanwälte mbB

(57) **Abstract**

A balloon catheter is provided with a main tube and an air tube therein. The main tube is provided with a central lumen to allow the balloon catheter to travel across and be guided along a guidewire during insertion into a vasculature of a patient. The air tube is configured to transfer fluid, such as air, to a balloon for inflating and deflating the balloon. The air tube can be embedded within the inner and outer surfaces of the main tube. Holes are cut into the main tube and a portion of the air tube to expose the air tube to the environment outside of the main tube. The holes allow for fluid communication between the air tube and the balloon. The holes are cut or sheared at a direction that is lateral to the longitudinal direction of the catheter tube.

## Description

The present disclosure relates generally to catheters, and more particularly to balloon catheters and methods of manufacturing the same.

### BACKGROUND

Various forms of balloon catheters are used in a wide range of medical procedures, such as percutaneous transluminal coronary angioplasty (PTCA), endovascular exclusion of abdominal aortic aneurysm (AAA) using stent grafts, and others. Balloon catheters can include two separate lumens, namely a guidewire lumen for traveling across a guidewire to and from a desired location within the artery, and a fluid lumen (e.g., air lumen) for delivering airflow to inflate the balloon. During a procedure, the balloon catheter is inserted and guided along the guidewire. The balloon catheter has an inflatable balloon at its tip. Once inserted, air can be pumped into the balloon to enlarge a narrow opening or passage within the vasculature.

### SUMMARY

According to one embodiment, a method of manufacturing a balloon catheter comprises: providing a fluid tube between an inner surface of a catheter tube and an outer surface of the catheter tube, wherein the fluid tube defines an fluid lumen therein extending along a longitudinal direction of the catheter tube; and shearing a portion of the catheter tube and the fluid tube to create a hole in the catheter tube that allows fluid communication between the fluid lumen to a balloon external to the outer surface of the catheter tube, wherein the shearing is performed in a direction lateral to the longitudinal direction of the catheter tube.

According to an embodiment, a method of manufacturing a balloon catheter comprises: providing a fluid tube between an inner surface of a catheter tube and an outer surface of the catheter tube, wherein the fluid tube defines a fluid lumen therein, and wherein the catheter tube extends along a central axis that lies on a bisecting plane; and shearing a portion of the catheter tube and the fluid tube to create a hole in the catheter tube that allows fluid communication between the fluid lumen and a balloon external to the outer surface of the catheter tube, wherein the shearing is performed in a direction parallel to the bisecting plane.

According to an embodiment, a balloon catheter comprises: a catheter tube having an inner surface and an outer surface, wherein the catheter tube defines a main lumen therein extending along a main axis, and wherein the catheter tube defines a hole therein that extends through the outer surface; and a fluid tube located between the inner surface of the catheter tube and the outer surface of the catheter tube, wherein the fluid tube defines a fluid lumen extending parallel to the main axis, and wherein the hole fluidly connects the fluid lumen to a balloon located outside the outer surface of the catheter tube; wherein a cross section of the catheter tube at the hole is in a circular segment shape.

Further disclosed herein is a balloon catheter with a main tube and an air tube therein, wherein the main tube is provided with a central lumen to allow the balloon catheter to travel across and be guided along a guidewire during insertion into a vasculature of a patient, wherein the air tube is configured to transfer fluid, such as air, to a balloon for inflating and deflating the balloon, wherein the air tube can be embedded within the inner and outer surfaces of the main tube, wherein holes are cut into the main tube and a portion of the air tube to expose the air tube to the environment outside of the main tube, wherein the holes allow for fluid communication between the air tube and the balloon, and wherein the holes are cut or sheared at a direction that is lateral to the longitudinal direction of the catheter tube.

### BRIEF DESCRIPTION OF THE DRAWINGS

Figure 1A is an illustration of a balloon catheter system, also referred to as a balloon on delivery catheter (BDC), according to an embodiment.
Figure 1B is an enlarged view of a balloon at a tip of the balloon catheter system, according to an embodiment.
Figure 2 is a cross-sectional view of a portion of the tip of the balloon catheter system where air enters the balloon to inflate the balloon, according to an embodiment.
Figure 3 is a cross-sectional view taken along line 3-3 of Figure 2 at a location in which one of the air holes is located, according to an embodiment.
Figure 4 is a top view of one of the air holes of the balloon catheter system, according to an embodiment.
Figure 5 is a side view of a portion of the balloon catheter system showing two of the air holes from the side, according to an embodiment.
Figure 6 is a perspective simplified geometrical view illustrating the plane in which a direction of cutting or shearing is located, according to an embodiment.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid and renal arteries, the invention may also be used in any other body passageways where it is deemed useful.

Various forms of balloon catheters are used in a wide range of medical procedures. Such procedures include, for example, angioplasty, such as percutaneous transluminal coronary angioplasty (PTCA), endovascular exclusion of abdominal aortic aneurysm (AAA) using stent grafts, and others. Balloon catheters can include two separate lumens, namely a guidewire lumen (also referred to as a main lumen) for traveling across a guidewire to and from a desired location within the artery, and a fluid lumen (e.g., air lumen) for delivering airflow to inflate the balloon. During a procedure, the balloon catheter is inserted and guided along the guidewire. The balloon catheter has an inflatable balloon at its tip. Once inserted, fluid such as air or saline can be pumped into the balloon to enlarge a narrow opening or passage within the vasculature.

During a process of endoluminal AAA repair using a stent graft (as an example), a balloon catheter can be used to appropriately seat the stent graft in a desired location within the vasculature. In general, stent grafts for treatment of vascular aneurysm and blood vessel walls which have been thinned or thickened by disease (endoluminal repair or exclusion). Many stents and stent grafts are "self-expanding" in that they may be inserted into the vasculature in a constricted configuration, and are biased to expand radially outwardly once an outer sheath containing the stent graft is removed. To avoid endoleaks, a balloon on a balloon catheter can be used to properly seat the stent-graft against the blood vessel wall or walls.

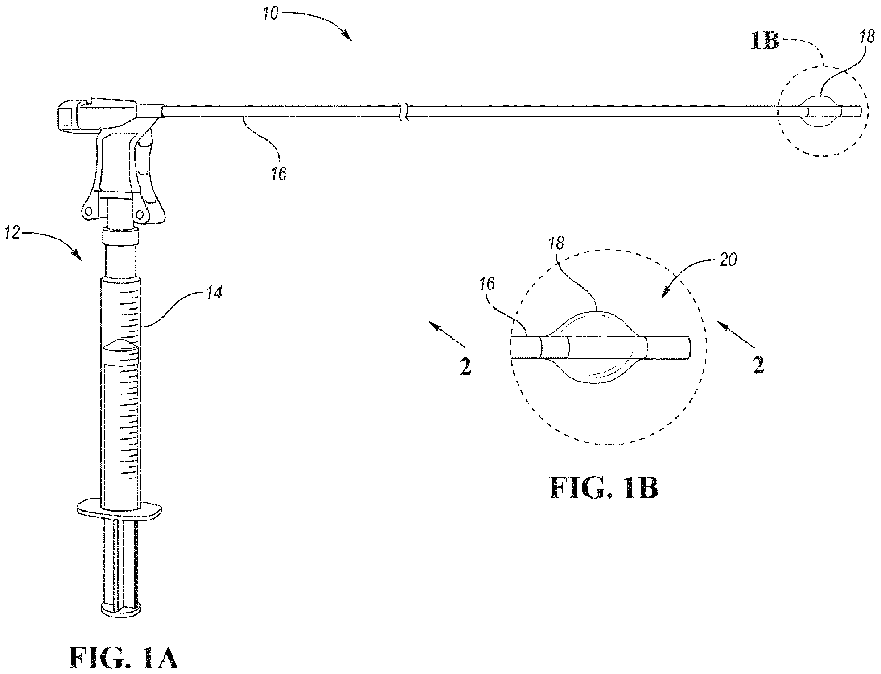

A balloon catheter 10 is shown in Figure 1A according to one embodiment. This illustration is but one example of a balloon catheter, and is not intended to be limited to only the illustrated embodiment. The balloon catheter 10 may include a balloon inflation device 12 configured to deliver a fluid, such as air or saline, to the balloon. The balloon inflation device 12 may include a syringe 14, piston, and/or other similar structure that can be manually manipulated by a surgical technician to pump or press fluid (e.g., saline, air) into a catheter tube 16. The catheter tube 16 has a lumen defined therein that is configured to deliver the fluid from the balloon inflation device 12 to dilatation balloon, generally referred to as a balloon 18. The balloon 18 is in fluid communication with the lumen within the catheter tube 16. As the fluid is delivered to the balloon 18, the balloon 18 inflates; as the fluid is retracted from the balloon 18, the balloon deflates. A surgical technician can thus control the size of the balloon 18 while inserted in a patient's vasculature by operating the balloon inflation device.

Figure 1B shows an enlarged portion of Figure 1A, namely the balloon 18. The balloon 18 is located at or around a distal tip 20 of the balloon catheter 10. As will be described, the catheter tube 16 of the balloon catheter 10 may include multiple tubes and/or lumens therein. For example, an inner tube (also referred to as a guidewire tube or main tube) may be provided that guides the balloon catheter 10 across a guidewire, and an outer tube (also referred to herein as an air tube) may be provided that defines a fluid lumen where the fluid transfers from the balloon inflation device 12 and into the balloon 18. The outer tube may be fluidly isolated from the inner tube such that one lumen is dedicated for travel over the guidewire, and another separate lumen is dedicated for the transfer of the fluid.

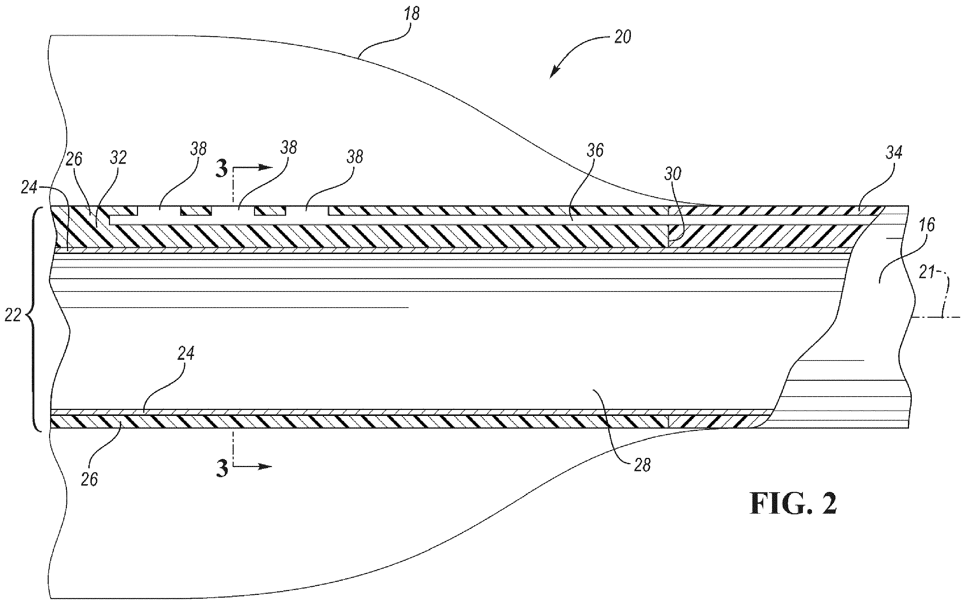

Figure 2 illustrates a cross-sectional view taken along line 2-2 of Figure 1B. Figure 2 shows the distal tip 20 of the balloon catheter 10 according to an embodiment. As mentioned before, the catheter tube 16 may include multiple tubes and/or lumens therein, which extend along or parallel to a central axis 21 of the catheter tube 16. In the illustrated embodiment, the catheter tube 16 includes a main tube 22 which itself has an inner layer 24 and an outer layer 26. The catheter tube 16 and main tube 22 may be one and the same tube structure; in other embodiments, the catheter tube 16 can refer to an outer sheath or tube placed radially about the main tube 22. The catheter tube 16 defines a main lumen 28 within the inner layer 24. The main lumen 28 is sized and configured to receive a guidewire such that the catheter tube 16 can slide along the guidewire to a desired location within the patient's vasculature.

The inner layer 24 may be annular in shape to define the main lumen 28 therein. The inner layer 24 may be made of a polymeric material, for example, a synthetic tetrafluoroethylene such as polytetrafluoroethylene (PTFE). The inner layer 24 may also be provided with a braid structure for structural rigidity and support. The braid may be made of metal, such as stainless steel, for example.

The outer layer 26, also referred to as an outer jacket, may be annular in shape and fully encapsulate or circumscribe the inner layer 24. The outer layer 26 may be made of a thermoplastic elastomer (TPE) such as a polyether block amide (PEBA). One example of PEBA is known under the tradename PEBAX. The outer layer may include two materials bonded together at a connection 30. In particular, the outer layer 26 may include a first material 32 made of TPE such as PEBA, and a second material 34 made of TPE such as PEBA. The first material 32 is located more towards the distal end of the balloon catheter 10 and therefore may be thicker and/or less rigid than the second material 34 to provide proper flexibility at the tip of the balloon catheter 10 which may be beneficial during insertion through the vasculature.

The balloon catheter 10 also includes a fluid tube, also referred to as an air tube 36. While references herein are made to "air" being delivered through the air lumen 36, it should be understood that any other suitable fluid - either liquid or gaseous - can be used to inflate the balloon depending on the particular device and medical procedure used. The structure described herein can be implemented in both an air balloon or a fluid (e.g., saline) balloon, and references to an air lumen or air balloon are not intended to be limited to requiring the use of air to inflate the balloon 18.

The air tube 36 defines an air lumen therein configured to deliver air to the balloon 18 from, e.g., the balloon inflation device 12. The air tube 36 may be made of a highperformance plastic or polymer, such as polyimide. The air tube 36 may be embedded within the outer layer 26 of the main tube 22, and may be at least partially circumscribed by the outer layer 26. In other embodiments, the air tube 36 is an air lumen formed by removing material from the outer layer 26 of the main tube 22. In other words, the air tube can be defined within the material of the main tube 22 rather than being a separate component embedded within the main tube 22.

At a distal end of the air tube 36 are a plurality of holes 38. The holes 38 allow the air from the air tube 36 to inflate the balloon 18. In other words, the holes 38 extend through a portion of the outer layer 26 and into air tube 36 to expose the air tube 36 to the environment outside of the main tube 22. Therefore, as air is forced into the air tube 36, it escapes the air tube 36 into the balloon 18 via the holes 38.

During manufacturing, the inner layer 24 and the air tube 36 may be joined together by lamination, overmolding, or co-molding of the outer layer 26. This embeds the air tube 36 within the outer layer 26. Due to this manufacturing process, the holes 38 are then later cut or formed into the outer layer 26. However, this can be challenging because cutting the holes 38 at a direction toward the center of the air tube 36 requires an extremely fine depth control so as to not pierce through to the main lumen 28. Additionally, direct cutting or piercing involves risks of the removed material becoming caught in the air tube 36, potentially causing a blockage.

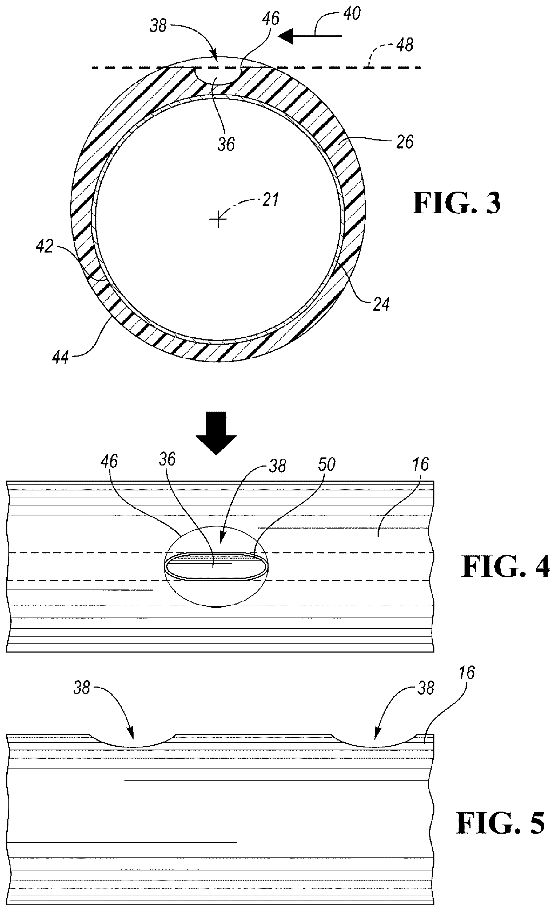

Therefore, according to various embodiments described herein, the holes 38 are formed by cutting in a lateral direction. Figure 3 shows a cross-sectional view taken along line 3-3 of Figure 2, and the cutting direction is shown by arrow 40. The cutting direction 40 is in a direction cross or lateral relative to the central axis 21, yet offset from (e.g., above) the axis 21. The holes 38 are not made by cutting down in a direction toward the interior of the main lumen 28, but are rather made by shearing off a portion of the outer layer 26 in a direction across the main tube 22. It can thus be said that the cutting direction 40 is generally parallel to a tangent of the main tube 22, or closer to being parallel to the tangent than perpendicular to the tangent.

Referring to Figure 3, a cross-section of the main tube 22 of the balloon catheter is shown. The inner layer 24 as explained above is annular and extends entirely about the central axis 21. During the cutting process described herein for creating the holes 38, the inner layer 24 is not cut. The outer layer 26 circumscribes the inner layer 24. The outer layer 26 has an inner surface 42 that directly contacts the inner layer 24. The outer layer 26 has an opposing outer surface 44. As can be seen in Figure 3, the shape of the outer layer 26 may be generally circular, but not a perfect circle. In particular, the outer layer 26 may be thicker (e.g., have more material) at one side of the central axis 21 than the other side, creating a lopsided layer. In the illustrated embodiment, the outer layer 26 is thicker at the side of the main tube 22 where the air tube 36 is located. In other words, the radial distance between the inner surface 42 and outer surface 44 is smaller at the bottom of the main tube 22 than at the top of the main tube 22. This provides additional material to support and surround the air tube 36.

Due to the direction of cutting at 40, the main tube 22 of the balloon catheter generally assumes a circular segment shape (although as described above, the outer layer 26 may not be a perfect circle). In particular, the cutting direction 40 creates cutting surfaces 46 on either lateral side of the air tube 36. The cutting surface 46 appear to be flat when viewing from a cross-sectional view as shown in Figure 3 due to the transverse direction of cutting along direction of arrow 40. All material of the main tube 22 is removed above the line 48 due to the transverse cutting, thus creating a circular segment shape (in the view of Figure 3) in which the outer layer 26 of the main tube 22 assumes a general circular shape except for the material "cut away" above the line 48. The depth of the cut may be selected such that line 48 intersects the air tube 36 in order to create a void or opening in the wall of the air tube 36. The void created by removing the material of the outer layer 26 above the line 48 creates the holes 38 that allow the air tube 36 to have fluid communication with the environment outside of the outer layer 26, e.g., the balloon 18.

Figure 4 illustrates a top view of the catheter tube 16, and Figure 5 illustrates a side view of the catheter tube 16, according to embodiments. A hole (e.g., hole 38) is formed in the catheter tube 16 from the shearing or cutting motion as described before. The cutting direction is shown again at 40. The same shearing or cutting that forms the hole 38 also cuts open a portion of the air tube 36. This leaves an open and exposed region of the air tube 36, allowing free communication of air to the balloon (not shown here) above the hole 38. Also, an exposed edge 50 of the air tube 36 is visible. The exposed edge 50 is a planar surface, coplanar with the cutting surfaces 46 on either lateral side of the air tube 36.

The transverse cutting - e.g., cutting across the length of the main tube 22 such as parallel to a tangent of the main tube 22 - can create uniform depth along the cutting direction. In other words, the height of the outer layer 26 can be uniform along any line that is parallel to the cutting direction. Referring to Figure 4, surfaces 46 and edge 50 are colinear along any line going up and down relative to the illustrated view.

The balloon catheter 10 can therefore be said to be manufactured as follows, according to an embodiment. The air tube can be embedded (laminated, overmolded, comolded, wrapped, etc.) within the outer layer 26 of the catheter tube 16 (or main tube 22), between the inner surface 42 of the catheter tube 16 and the outer surface 44 of the catheter tube 16. Thereafter, an operator or machinery can cut or shear a portion of both the catheter tube 16 and the air tube 36 along a direction 40 that is lateral to the longitudinal direction of the catheter tube 16.

The cutting that removes the material above line 48 can be performed by any number of suitable tools. In one embodiment, a drill cutter having a rotating bit with a sharpened point can be activated to spin and press into the outer layer 26 to remove the material. In other embodiments, blades or the like can be used to cut a straight, flat cutting edge.

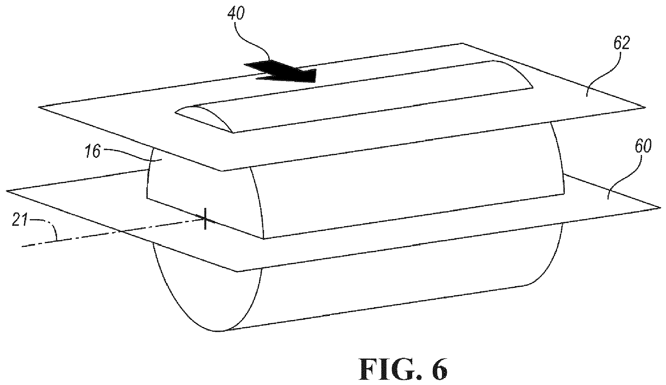

Referring to Figure 6, a geometrical representation of the cutting or shearing direction is illustrated with respect to a three-dimensional simplified illustration of the catheter tube 16. This embodiment is simplified for geometry explanation. A first plane 60 and a second plane 62 are shown. The first plane 60 bisects (or roughly bisects) the catheter tube 16. The central axis 21 lies on the first plane 60. The second plane 62 is parallel to the first plane 60. The direction of cutting 40 lies on the second plane 62.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

Further disclosed herein is the subject-matter of the following clauses:
1. A method of manufacturing a balloon catheter, the method comprising:
   providing a fluid tube between an inner surface of a catheter tube and an outer surface of the catheter tube, wherein the fluid tube defines a fluid lumen therein extending along a longitudinal direction of the catheter tube; and
   shearing a portion of the catheter tube and the fluid tube to create a hole in the catheter tube that allows fluid communication between the fluid lumen to a balloon external to the outer surface of the catheter tube, wherein the shearing is performed in a direction lateral to the longitudinal direction of the catheter tube.
2. The method of clause 1, wherein the direction of the shearing is such that it does not intersect the inner surface of the catheter tube.
3. The method of clause 1 or of any of the preceding clauses, wherein the direction of the shearing is parallel to a tangent of the catheter tube.
4. The method of clause 1 or of any of the preceding clauses, wherein the catheter tube includes an inner layer and an outer layer, and wherein the shearing of the portion of the catheter tube includes shearing of only the outer layer and not the inner layer.
5. The method of clause 1 or of any of the preceding clauses, wherein the step of providing includes embedding the fluid tube within the catheter tube.
6. The method of clause 5, wherein the catheter tube has an inner layer and an outer layer, and wherein the embedding includes embedding the fluid tube within the outer layer of the catheter tube.
7. A method of manufacturing a balloon catheter, the method comprising:
   providing a fluid tube between an inner surface of a catheter tube and an outer surface of the catheter tube, wherein the fluid tube defines a fluid lumen therein, and wherein the catheter tube extends along a central axis that lies on a bisecting plane; and
   shearing a portion of the catheter tube and the fluid tube to create a hole in the catheter tube that allows fluid communication between the fluid lumen and a balloon external to the outer surface of the catheter tube, wherein the shearing is performed in a direction parallel to the bisecting plane.
8. The method of clause 7, wherein the direction of the shearing is such that it does not intersect the inner surface of the catheter tube.
9. The method of clause 7 or of any of clauses 7-8, wherein the direction of the shearing is parallel to a tangent of the catheter tube.
10. The method of clause 7 or of any of clauses 7-9, wherein the catheter tube includes an inner layer and an outer layer, and wherein the shearing of the portion of the catheter tube includes shearing of only the outer layer and not the inner layer.
11. The method of clause 7 or of any of clauses 7-10, wherein the step of providing includes embedding the fluid tube within the catheter tube.
12. The method of clause 11, wherein the catheter tube has an inner layer and an outer layer, and wherein the embedding includes embedding the fluid tube within the outer layer of the catheter tube.
13. A balloon catheter comprising:
   a catheter tube having an inner surface and an outer surface, wherein the catheter tube defines a main lumen therein extending along a main axis, and wherein the catheter tube defines a hole therein that extends through the outer surface; and
   a fluid tube located between the inner surface of the catheter tube and the outer surface of the catheter tube, wherein the fluid tube defines a fluid lumen extending parallel to the main axis, and wherein the hole fluidly connects the fluid lumen to a balloon located outside the outer surface of the catheter tube;
   wherein a cross section of the catheter tube at the hole is in a circular segment shape.
14. The balloon catheter of clause 13, wherein the circular segment shape is round except for a region of the fluid tube where the fluid hole is in fluid communication with the balloon.
15. The balloon catheter of clause 14, wherein a portion of material of the catheter tube is removed on either lateral side of the air hole at the cross-section.
16. The balloon catheter of clause 13 or of any of clauses 13-15, wherein the circular segment shape has a flat surface on either later side of the hole, wherein the flat surfaces are colinear.
17. The balloon catheter of clause 13 or of any of clauses 13-16, wherein the cross section of the fluid tube has a lower region and an opposing upper region, wherein the lower region is thinner than the upper region.
18. The balloon catheter of clause 17, wherein the hole is located in the upper region.
19. The balloon catheter of clause 13 or of any of clauses 13-18, wherein the catheter tube includes an inner layer of material and an outer layer of material, wherein the fluid tube is embedded within the outer layer of material.
20. The balloon catheter of clause 19, wherein the inner layer of material includes polytetrafluoroethylene (PTFE) and the outer layer of material includes polyether block amide (PEBA).

## Claims

1. A method of manufacturing a balloon catheter, the method comprising:
providing a fluid tube between an inner surface of a catheter tube and an outer surface of the catheter tube, wherein the fluid tube defines a fluid lumen therein extending along a longitudinal direction of the catheter tube; and
shearing a portion of the catheter tube and the fluid tube to create a hole in the catheter tube that allows fluid communication between the fluid lumen to a balloon external to the outer surface of the catheter tube, wherein the shearing is performed in a direction lateral to the longitudinal direction of the catheter tube.

2. The method of claim 1, wherein the direction of the shearing is such that it does not intersect the inner surface of the catheter tube.

3. The method of any of the preceding claims, wherein the direction of the shearing is parallel to a tangent of the catheter tube.

4. The method of any of the preceding claims, wherein the catheter tube includes an inner layer and an outer layer, and wherein the shearing of the portion of the catheter tube includes shearing of only the outer layer and not the inner layer.

5. The method of any of the preceding claims, wherein the step of providing includes embedding the fluid tube within the catheter tube.

6. The method of claim 5, wherein the catheter tube has an inner layer and an outer layer, and wherein the embedding includes embedding the fluid tube within the outer layer of the catheter tube.

7. A method of manufacturing a balloon catheter, the method comprising:
providing a fluid tube between an inner surface of a catheter tube and an outer surface of the catheter tube, wherein the fluid tube defines a fluid lumen therein, and wherein the catheter tube extends along a central axis that lies on a bisecting plane; and
shearing a portion of the catheter tube and the fluid tube to create a hole in the catheter tube that allows fluid communication between the fluid lumen and a balloon external to the outer surface of the catheter tube, wherein the shearing is performed in a direction parallel to the bisecting plane.

8. The method of claim 7, wherein the direction of the shearing is such that it does not intersect the inner surface of the catheter tube, and/or wherein the direction of the shearing is parallel to a tangent of the catheter tube, and/or wherein the catheter tube includes an inner layer and an outer layer, and wherein the shearing of the portion of the catheter tube includes shearing of only the outer layer and not the inner layer.

9. The method of any of claims 7-8, wherein the step of providing includes embedding the fluid tube within the catheter tube, wherein typically the catheter tube has an inner layer and an outer layer, and wherein the embedding includes embedding the fluid tube within the outer layer of the catheter tube.

10. A balloon catheter comprising:
a catheter tube having an inner surface and an outer surface, wherein the catheter tube defines a main lumen therein extending along a main axis, and wherein the catheter tube defines a hole therein that extends through the outer surface; and
a fluid tube located between the inner surface of the catheter tube and the outer surface of the catheter tube, wherein the fluid tube defines a fluid lumen extending parallel to the main axis, and wherein the hole fluidly connects the fluid lumen to a balloon located outside the outer surface of the catheter tube;
wherein a cross section of the catheter tube at the hole is in a circular segment shape.

11. The balloon catheter of claim 10, wherein the circular segment shape is round except for a region of the fluid tube where the fluid hole is in fluid communication with the balloon, wherein typically a portion of material of the catheter tube is removed on either lateral side of the air hole at the cross-section.

12. The balloon catheter of any of claims 10-11, wherein the circular segment shape has a flat surface on either later side of the hole, wherein the flat surfaces are colinear.

13. The balloon catheter of any of claims 10-12, wherein the cross section of the fluid tube has a lower region and an opposing upper region, wherein the lower region is thinner than the upper region, wherein typically the hole is located in the upper region.

14. The balloon catheter of any of claims 10-13, wherein the catheter tube includes an inner layer of material and an outer layer of material, wherein the fluid tube is embedded within the outer layer of material.

15. The balloon catheter of claim 14, wherein the inner layer of material includes polytetrafluoroethylene (PTFE) and the outer layer of material includes polyether block amide (PEBA).
